# EUROPEAN PATENT SPECIFICATION

(11) **EP 2 763 468 B1**
(45) Date of publication and mention of the grant of the patent: **30.08.2017**
(21) Application number: 13405022.8
(22) Date of filing: 31.01.2013
(51) Int. Cl.: H04W 52/02, G01N 27/12, G01N 33/497, G01N 33/00

(54) **Portable sensor device with a gas sensor and method for operating the same**
Tragbare Sensorvorrichtung mit einem Gassensor und Verfahren zum Betrieb davon
Dispositif de capteur portable avec un capteur de gaz et son procédé de fonctionnement

(43) Date of publication of application: 06.08.2014
(73) Proprietor: Sensirion AG, 8712 Stäfa (CH)
(72) Inventor: Schmidlin, Roger, 8053 Zürich (CH); Bühler, Johannes, 8610 Uster (CH)
(74) Representative: Sutter, Kurt

(56) References cited:
- US-A1- 2005 101 250
- US-A1- 2012 270 611
- Michael Karst ET AL: "Humidity & Temperature Sensors in Mobile Phones", Wireless Communication Alliance (WCA) event , 18 April 2012 (2012-04-18), pages 1-18, XP055069470, Retrieved from the Internet: URL:http://www.wca.org/event_archives/2012 /Sensirion_WCA_April2012_Mobile_Sensors.pd f [retrieved on 2013-07-03]
- ANAND D MANE ET AL: "Explosive detection with mobile telephony an attempt towards a safe ambience", PROCEEDINS OF THE 2011 IEEE INTERNATIONAL CONFERENCE ON SIGNAL PROCESSING, COMMUNICATION, COMPUTING AND NETWORKING TECHNOLOGIES (ICSCCN), 21 July 2011 (2011-07-21), pages 187-191, XP031940738, DOI: 10.1109/ICSCCN.2011.6024541 ISBN: 978-1-61284-654-5

## Description

### Technical Field

The invention relates to a method for operating a portable sensor device having a gas sensor as well as to a device of this type. In addition, the method relates to a control and processing means to be used in such a device.

### Background Art

It has been known to incorporate gas sensors into portable sensor devices, such as mobile phones or tablet computers. For example, humidity sensors have been incorporated into some smartphone devices.

The pdf document by Michael Karst et al. as retrievable from http://www.wca.org/event archives/2012/Sensirion WCA April2012 Mobile Sensors.pdf discloses humidity and temperature sensors for mobile phones.

US 2012/270611 A1 discloses a method for controlling a mobile terminal.

To expand the versatility of portable electronic devices, it is further known to provide a mechanism for running third party software applications on these devices. For this purpose, the devices are provided with an application programming interface (API), which allows the applications to communicate with the device's hard- and software in a standardized manner. If the device is equipped with a gas sensor, that API should also allow access to the gas sensor.

An example of an API for a gas sensor is the Environment Sensors API of the Android operating system. In particular, this API supports humidity sensors, which measure the humidity of the ambient air.

However, humidity (i.e. gaseous water in air) is only one gas that might be of interest to a user of this type of device. Therefore, there is a need to provide devices that allow to measure a larger selection of gases, such as alcohol, CO, benzene, or groups of gases, which can e.g. be classified as certain smells or odours.

Even though there are various sensor types, in particular metal-oxide gas sensors, that allow to detect such gases or groups of gases these sensors are typically not very selective and are more or less sensitive to whole classes of gases. In order to distinguish between certain gases or groups of gases, a plurality of sensors may have to be combined and/or a sensor must be operated in a certain fashion, e.g. using a certain temperature profile.

The development of such gas sensors and the methods for operating the same is still in flux, and substantial progress is to be expected in the next years. However, there is a need to incorporate such sensors into mobile devices right now and to provide an API for them.

### Disclosure of the Invention

Therefore, the problem to be solved is to provide a method, device and control and processing means of the type above that allows to be used with novel and improved gas sensors.

This problem is solved by the subject matter of the independent claims.

In particular, the invention relates to a method for operating a portable sensor device, which portable sensor device comprises
a gas sensor,
a control and processing means for controlling said gas sensor and for processing data from said gas sensor, and
an application programming interface means that provides access to said control and processing means.

The method comprises the following steps:
a) Providing a list of a plurality of different operating modes and providing said list to the application programming interface means. Each of said operating modes is indicative of a method for controlling said gas sensor and/or processing said data from said gas sensor. At least some of the operating modes are indicative of different methods for controlling said gas sensor. Alternatively or in addition thereto, at least some of said operating modes are indicative of different methods for processing the data from the gas sensor.
b) Receiving a selection, made through said application programming interface means, of one of said operating modes, and controlling the gas sensor and/or processing the data from the gas sensor according to the selected operating mode.

In other words, the software underlying the API is capable to generate a list of the different operating modes. Each operating mode defines a certain way to control the gas sensor and/or a certain way to process the signal(s) from the gas sensor.

The software application calling the API is able to obtain and parse the list. It then selects a suitable operating mode through the API. The underlying software receives this selection and controls the gas sensor and/or processes the data from the gas sensor in accordance with the selected operating mode.

Similarly, the invention relates to a portable sensor device comprising
a gas sensor,
a control and processing means for controlling the gas sensor and for processing data from the gas sensor,
an application programming interface means that provides access to the control and processing means.

Further, the control and processing means comprises
an operating mode list provider for providing a list of a plurality of different operating modes and providing said list to said application programming interface means, wherein each of said operating modes is indicative of a method for controlling the gas sensor and/or processing the data from the gas sensor, and
an operating mode selector adapted to receive a selection of an operating mode through said application programming interface means and to control the gas sensor and/or to process the data from the gas sensor.

Again, at least some of the operating modes are indicative of different methods for controlling said gas sensor. Alternatively or in addition thereto, at least some of said operating modes are indicative of different methods for processing the data from the gas sensor.

Also, the invention relates to a control and processing means for being used in the method or the portable sensing device above, which control and processing means comprises
an operating mode list provider for providing a list of a plurality of different operating modes and providing said list to an application programming interface means, wherein each of said operating modes is indicative of a method for controlling said gas sensor and/or for processing said data from said gas sensor, and
an operating mode controller responsive to a selection of an operating mode made through said application programming interface means in order to control said gas sensor and/or to process said data from said gas sensor in accordance with the selected operating mode. At least some of the operating modes are indicative of different methods for controlling said gas sensor. Alternatively or in addition thereto, at least some of said operating modes are indicative of different methods for processing the data from the gas sensor.

The control and processing means is advantageously implemented as a software library, which comprises a piece of code that can be dynamically or statically linked to other parts of the operating system. In particular, the control and processing means can be a dynamically linked library (DLL).

As stated above, at least some of the operating modes are indicative of different methods for controlling said gas sensor. In particular, different operating modes can designate different temperatures or temperature profiles under which the gas sensor is to be operated. For example, one such profile may select the gas sensor to be operated at a first temperature, while a second profile may select the gas sensor to be operated at a second temperature, different from the first temperature.

As stated above, alternatively or in addition thereto, at least some of said operating modes are indicative of different methods for processing the data from the gas sensor. For example, one operating mode may indicate that the data from the gas sensor is to be normalized to a reference value or filtered with a low-pass filter, while another one may indicate that non-normalized data or unfiltered data is to be returned.

The mobile device may also comprise a second sensor, in addition to the gas sensor. The second sensor may for example be a humidity sensor. In that case, at least one of said operating modes can be indicative of combining, in above step b), data from the second sensor with data from the gas sensor, while at least another one of the operating modes is indicative of not combining, in step b), the data from the second sensor with data from the gas sensor. In that case, when selecting one operating mode, the operating mode controller combines data from both sensors, while, when selecting the other operating mode, it doesn't.

The mobile device may also comprise a plurality of gas sensors, in particular of metal-oxide gas sensors. In that case, at least some of the operating modes can be indicative of differently using the signals from these gas sensors in step b). In other words, depending on the selected operating mode, the signals from at least two or more gas sensors may e.g. be combined, while in another selected operating mode, they aren't.

Advantageously, the sensor comprises a metal-oxide layer and a heater for heating said metal-oxide layer. This type of sensor can be used to detect a variety of gases.

In yet another advantageous embodiment, the operating modes can be grouped into at least two groups:
- A first group of the operating modes is designed to detect individual gases or gas mixtures and to generate a measurement value indicative of a presence and/or concentration of said individual gas or gas mixture. In other words, one gas or gas mixture is attributed to each operating mode in this group. For example, the first group can comprise operating modes optimized for detecting ethanol, CO, or "gases with unpleasant smell". Selecting an operating mode from this group will make the system return a value that indicates if the gas or group gases attributed to the selected mode is present and/or what its concentration is.
- A second group of the operating modes is designed to analyze a gas composition of several constituents and to return measurement values indicative of a presence and/or concentration of each of said constituents. For example, the second group may comprise an operating mode called "gas analysis", which returns an array of values that represents a "fingerprint" of the presently measured gas composition that varies depending on the gas composition.

It must be noted that the present invention can be viewed as method, a device or a control and processing means, as claimed. In particular, any of its features can be claimed as a method, a device or a control and processing means, and it is apparent that any features formulated in the claims under one of these three categories can also be formulated in the claims under another one of these three categories.

Other advantageous embodiments are listed in the dependent claims as well as in the description below.

### Brief Description of the Drawings

The invention will be better understood and objects other than those set forth above will become apparent from the following detailed description thereof. Such description makes reference to the annexed drawings, wherein:
Fig. 1 is a perspective view of a portable electronic sensor device with a gas sensor,
Fig. 2 is a block diagram of hardware components of the device,
Fig. 3 shows a gas sensor in more detail,
Fig. 4 is a block diagram of software components of the device, and
Fig. 5 is a block diagram of some components of the control and processing means for the gas sensor.

### Modes for Carrying Out the Invention

### Device Hardware:

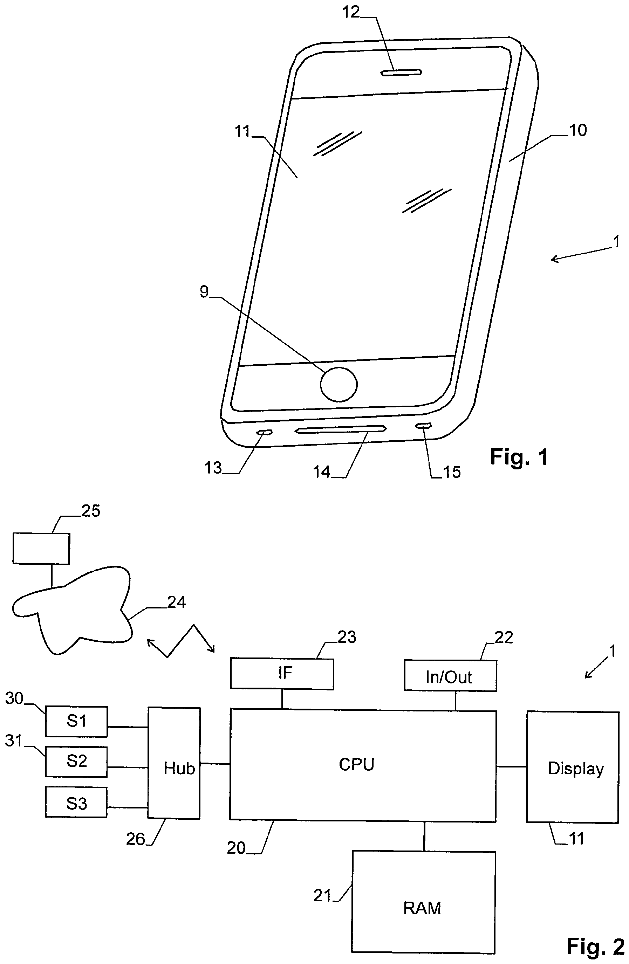

The sensor device of Fig. 1 is a portable electronic device 1, such as a mobile phone. The housing 10 of the mobile phone includes a front side with a display 11 and input elements like buttons 9 to let a user interact with the phone. Also shown on the front side is an opening 12 for a loudspeaker. Further openings 13, 14 are located at a lower side wall of the housing 10. It is well known to mount components like microphones and loudspeakers behind such openings. A further opening 15, which is e.g. also arranged on the lower side wall of housing 10, provides access to a gas sensor as described in more detail below.

Fig. 2 shows a block diagram with the most important components of the device. In particular, the device comprises a CPU 20 and non-volatile as well as volatile memory 21 as known to the skilled person. Fig. 2 also shows the display 11 and a group 22 of further input- and output devices, such as the button 9, the loudspeaker and the microphones.

Further, the device comprises a network interface 23, which is capable to establish wireless data communication with an external network 24, such as the internet. This network is connected to further devices. At least one server device 25 of these further devices is adapted to communicate with device 1 through network interface 23.

Device 1 further comprises a hub or bus 26 through which CPU 20 is able to communicate with a series of sensors S1, S2, S3... These sensors can e.g. comprise an accelerometer, one or more temperature sensors, and more. In particular, one of the devices is a gas sensor 30. In an advantageous embodiment, a further sensor is a humidity sensor 31, such as described in US 6 690 569.

### Gas sensor:

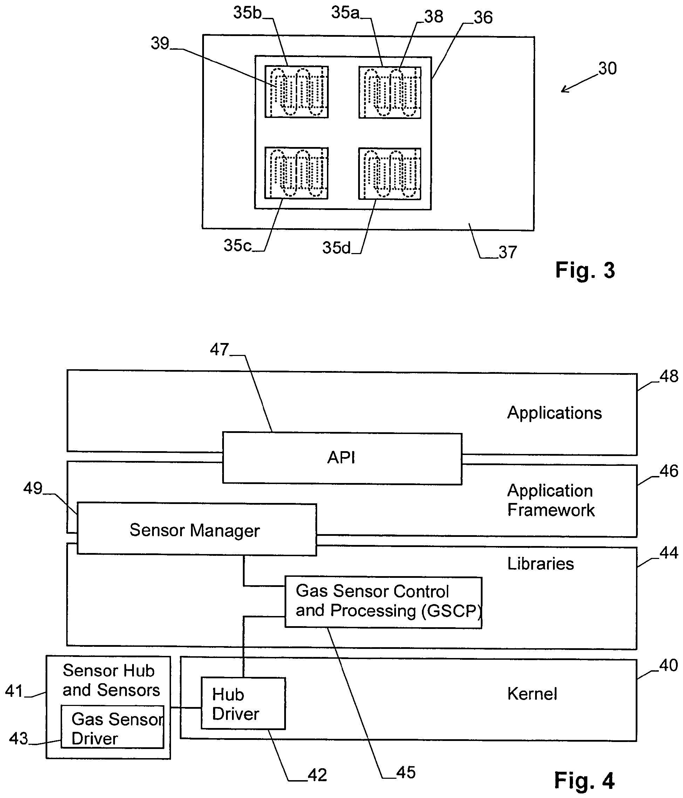

Fig. 3 shows an embodiment of a gas sensor 30. The shown sensor is e.g. a sensor basically of the type e.g. as described in WO 95/19563, where a sensing layer 35a, 35b, 35c, 35d, in particular of a metal-oxide, is arranged on a thin membrane 36 extending over an opening in semiconductor substrate 37.

In the embodiment of Fig. 3, there are four sensing layers 35a, 35b, 35c, 35d separately arranged on membrane 36, each of which forms a gas sensor of its own. The various sensing layers 35a, 35b, 35c, 35d can e.g. differ in their composition in order to measure different gas properties, thereby providing a richer data set for identifying individual analytes.

As mentioned, the sensing layers are advantageously metal-oxide (MOX) layers, such as layers of SnO. The MOX can also e.g. be tungsten oxide, gallium oxide, indium oxide, or zinc oxide, or a mixture of any of these materials, including SnO.

The sensing layers of the sensor of Fig. 3 require elevated temperatures for operation, typically of at least 100°C, for SnO-layers typically of at least 300°C. Hence, heaters 38 are provided for heating each of the sensing layers to its operating temperature.

As known to the skilled person, the conductance of the sensing layer 35a, 35b, 35c, 35d depends on the composition of the gas that surrounds it. Hence, interdigital electrodes 39 are provided for measuring the resistivity of the sensing layer 35a, 35b, 35c, 35d.

### Device software:

Fig. 4 shows the - for the present context - most relevant components of the software stack of the device. As can be seen, the software stack comprises a kernel 40 adapted to provide low-level functionality. The kernel e.g. comprises the individual device drivers adapted to interact with individual hardware components of the device.

In the embodiment of Fig. 4, sensor hub 26 is a programmable microcontroller that runs its own kernel software 41, which interacts with a hub driver 42 of the main kernel 40. The driver 43 for driving the gas sensor 30 is implemented in the hub kernel software 41. It must be noted, though, that driver 43 could also be implemented in the main kernel 40.

A library level 44 sits on top of kernel 40. It comprises a number of libraries, with each library providing functionality that is, at least to a certain degree, typically machine independent (in contrast to the kernel software which is typically adapted to the hardware of the device where it is running). As known to the skilled person, the operating system's runtime is typically implemented in at least one of these libraries.

Each library comprises typically one or more code files comprising code that can be dynamically or statically linked to other libraries or to applications. Typically, libraries are implemented as dynamically linked libraries (DLLs).

One library in library level 44 is the gas sensor control and processing library (GSCP) 45. Its purpose is to control the operation of gas sensor 30 and to process its signals. It will be described in more detail below.

On top of library level 44 sits the application framework 46, which is typically also implemented as a set of libraries. In contrast to most of the libraries in library level 44, the libraries of the application framework 46 provide a public interface 47 available to the topmost software level, the applications 48. The public interface 47 is called the Application Programming Interface API and it comprises calling standards (such as header files defining the available functions, classes and methods) as well as binary data (which is part of the libraries in the application framework) that indicates how an application can be dynamically linked to the individual functions and methods within the application framework.

In the present embodiment, part of the application framework is a sensor manager 49, which defines the part of the API that relates to the sensors of device 1 and which interacts with the sensor-related libraries and drivers in libraries level 44 or in kernel 40.

The applications 48 are typically provided by third parties (i.e. neither by the hardware manufacturer nor by the provider of the operating system). They link against the libraries laid open in the API on order to execute specific tasks.

For example, one such application may be an application that is supposed to detect a certain gas or to analyse the composition of the gases in contact with gas sensor 30. Such an application would use the sensor manager's 49 API in order to interact with gas sensor 30.

### Gas Sensor Control and Processing:

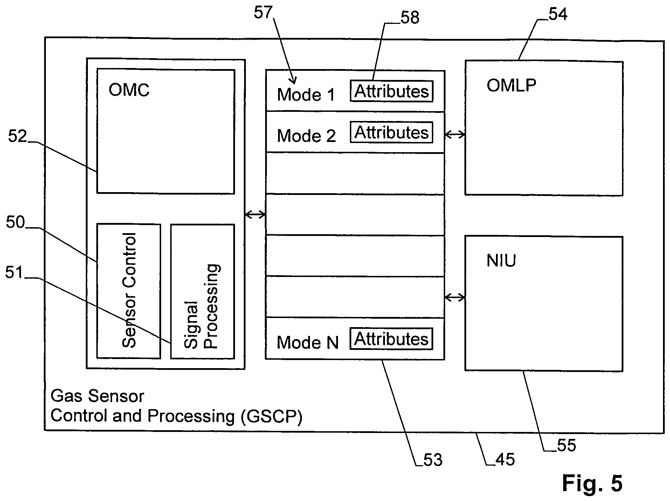

Gas sensor control and processing library GSCP (also called "control and processing means") 45 can be implemented in hard- or software or a mixture thereof. Advantageously, as mentioned, it is a software library. Fig. 5 shows the design of Gas sensor control and processing library GSCP 45 in more detail. It comprises the following components:
- A sensor controller 50 controls the operation of gas sensor 30. In particular, it is adapted to e.g. control the current through the heaters 38, e.g. in order to perform the measurement at a certain temperature or to use a certain time-dependent temperature profile. It can also be adapted to optionally control other sensors, such as humidity sensor 31. Sensor controller 50 interfaces with gas sensor driver 43 through hub driver 42.
- A signal processor 51 is adapted to receive the measured signals from gas sensor 30 (and, optionally, from further sensors, such as humidity sensor 31), and to process them in order to obtain a result data set. Signal processor 51 interfaces with sensor manager 49 in order to provide the result at API 47.
- An operating mode controller (OMC) 52 is provided for controlling the operation of sensor controller 50 and signal processor 51 depending on a currently selected operating mode. This operating mode defines the conditions under which gas sensor 30 is run and the algorithms and parameters used for processing the measured data in order to convert it to the result data set. Operating mode controller 52 interfaces with sensor manager 49 for receiving the selected operating mode through API 47.
- An operating mode table 53 containing a listing of all available operating modes.
- An operating mode list provider OMLP 54 for providing a list of the operating modes in operating mode table 53 to the API 47. For this purpose, OMLP 54 interfaces with sensor manager 49, through which this list is made available at API 47.
- GSCP 45 can contain further components for additional functionality. For example, it may contain a Network Interfacing Unit 55 (NIU) that interfaces with network 24 and in particular with server device 25. It can e.g. be used to obtain real-time data from server device 25 or to offload data processing to server device 25, functionalities which may be required for some of the operating modes in table 53.

As can be seen, table 53 contains, for each operating mode, an identifier 57 (such as "Mode 1", "Mode 2", etc. in the embodiment of Fig. 5) as well as attributes 58. The attributes can e.g. include the following information:
- A human-readable name of the operating mode (such as "detection of ethanol", "bad smell detection", "gas analysis").
- Parameters controlling sensor controller 50, thereby specifying one of various different methods for controlling gas sensor 30. In particular, these parameters can e.g. indicate the temperature or temperature profile under which the measurement is to take place.

- Parameters controlling signal processor 51. These parameters can e.g. indicate the algorithm to be used for processing the data from gas sensor 30 and/or the numerical parameters for doing so.
- An indicator describing the type of result returned through the API when the gas sensor is operated in the given operating mode. For example, the result may be a scalar, an array or an object.
- An indicator indicating if resources over network 24 are required for executing the measurement in the given operating mode, i.e. if access to remote resources is required through NIU 55.
- An indicator indicating the group of operating modes that the operating mode belongs to, as described with reference to "first group" and "second group" above. This indicator may also specify a subgroup. For example, if the operating method belongs to a group of methods identifying individual gases, the subgroup may specify the gas the operating method is designed for.
- An expected duration of the measurement in the given operating mode.

### The API:

In the following, an example of an API is provided in a java-like syntax similar to the one provided by the SensorManager class of the Android(R) operating system. The skilled person will understand, though, that the principles described in the following are in no way limited to a given operating system or programming language.

For example, it is assumed that API 47 is implemented an extension of the present definition of the class Sensor of the Andriod API.

In a first step, an application has to obtain an instance of the Sensor:
*mgr* = *(SensorManager) getSystemService(SENSOR_SERVICE) ;*
*sens* = *mSensorManager.getDefaultSensor(Sensor. TYPE_GAS) ;*

In a next step, the application can obtain a list of the available operating modes of the gas sensor:
*modes* = *sens.getModes( ) ;*

With getModes() e.g. being defined as follows:
*public HashMap<String,Object>[ ] getModes( ) ;*

This function returns an array of objects of type HashMap, with each HashMap being a key-value dictionary describing an operating mode. This dictionary is generated by operating mode list provider OMLP 54 of the gas sensor control and processing means GSPC 45.

For example, each dictionary can contain the following keys and values:

| | |
|---|---|
| key | value |
| "NAME" | The identifier of the operating mode, such as "Mode 1" in the example above |
| "DESCR" | A string with a human-readable description, such as "detection of ethanol" |
| "RESULT" | An integer number describing the type of the value returned as a result through the API when the sensor is operated in the given operating mode, e.g. 1 if the number is a scalar, scalar, a number N > 1 if the result is an array of length N, or 0 if the result is a java object. |
| "NETW" | A Boolean value indicative if the given operating mode requires network access for being executed. |
| "GROUP" | A string value indicative of the group that the operating mode belongs to, e.g. "SINGLE" if the operating mode is designed to detect a single gas, or "COMPOSITION" if the operating mode is designed to analyze the composition of a gas with several constituents. |
| "SUBGROUP" | A string value further specifying a subgroup as described above. For example, if the operating method belongs to the group "SINGLE", the subgroup can e.g. specify the name of the gas or gas mixture that the operating mode is optimized for, such as "ETHANOL", "CO", "SWEAT SMELL" etc. |
| "DURATION" | A floating point value describing an expected duration for a measurement, e.g. in seconds. |

Further keys and values can e.g. describe a numerical range (e.g. 0.0 ... 1.0) or scaling (linear, logarithmic) of the values to be returned, etc.

Once the application has obtained the list of operating modes, it can iterate through them to identify the most suitable operating mode.

Then, the application invokes a method of the Sensor class that specifies the operating mode to be used. This method may e.g. be defined as
*public void setSensorMode(string mode);*
and is to be invoked using the identifier of the desired operating mode in the parameter mode.

Once the operating mode is selected, measurements can be taken e.g. by installing a listener as provided by Android's SensorManager class:
*mgr.registerListener(this, sens,*
*SensorManager.SENSOR_DELAY_NORMAL)*

Results will then be sent to the method onSensorChanged() of the object "this". These results can then be processed depending on their type.

### Notes:

As mentioned, the above is only an example of an implementation of an API. The details of the specific API can vary.

For example, in the above example the list of different operating modes have been provided as an array of HashMap objects. The skilled person knows other methods to provide such a list, e.g. through List objects or by means of a callback function to be called for each item in the list.

Also, a mechanism is advantageously provided for avoiding collisions between operating modes selected by different, concurrently running applications. For example, one application may request GSCP 45 to operate in one operating mode, while another application wants to operate it in another processing mode. For such cases, for example, GSCP 45 may implement a time-sharing system to operate alternatingly in the two operating modes when it receives two different selections of operating modes from different sources.

In summary, in order to allow application programs access to a gas sensor, a versatile mechanism is provided that allows to provide a flexible sensor control through a simple, fixed API. The mechanism is designed to let the application program query the possible operating modes of the gas sensor. The operating modes e.g. specify the methods for controlling the gas sensor and for processing its raw data.

While there are shown and described presently preferred embodiments of the invention, it is to be distinctly understood that the invention is not limited thereto but may be otherwise variously embodied and practised within the scope of the following claims.

## Claims

1. A method for operating a portable sensor device, wherein said portable sensor device comprises
a gas sensor (30),
a control and processing means (45) for controlling said gas sensor (30) and for processing data from said gas sensor (30), and
an application programming interface means API (47) that provides access to said control and processing means (45),
said method being **characterized by** the steps of
a) providing a list of a plurality of different operating modes and providing said list through said API,
- wherein each of said operating modes is indicative of a method for controlling said gas sensor (30) and wherein at least some of said operating modes are indicative of different methods for controlling said gas sensor (30) and/or
- wherein each of said operating modes is indicative of a method for processing said data from said gas sensor (30) and wherein at least some of said operating modes are indicative of different methods for processing said data from said gas sensor (30), and
b) receiving a selection of one of said operating modes, made through said application programming interface means (47), and controlling said gas sensor (30) and/or processing said data from said gas sensor (30) according to the selected operating mode.

2. The method of any of the preceding claims, wherein at least some of said operating modes require access of the sensor device to resources (25) over a network (24), wherein said list comprises, for each operating mode, an indicator if said access is required.

3. The method of any of the preceding claims wherein said list is indicative of an expected duration for a measurement in each operating mode.

4. The method of any of the preceding claims wherein at least some of said operating modes are indicative of different temperatures or temperature profiles under which the gas sensor (30) is to be operated.

5. The method of any of the preceding claims, wherein said mobile device comprises a second sensor (31), in addition to said gas sensor (30), wherein at least one of said operating modes is indicative of combining, in said step b), data from said second sensor (31) with data from said gas sensor (30), wherein, at least another one of said operating modes is indicative of not combining, in said step b), the data from said second sensor (31) with data from said gas sensor (30).

6. The method of claim 5 wherein said second sensor (31) is a humidity sensor.

7. The method of any of the preceding claims, wherein said mobile device comprises a plurality (35a, 35b, 35c, 35c) of gas sensors, in particular of metal-oxide gas sensors, wherein at least some of said operating modes are indicative of differently using signals from said plurality (35a, 35b, 35c, 35c) of gas sensors in said step b).

8. The method of any of the preceding claims wherein
a first group of said operating modes is designed to detect individual gases or gas mixture and to generate a measurement value indicative of a presence and/or concentration of said individual gas or gas mixtures, while
a second group of said operating modes is designed to analyze a gas composition of several constituents and to return measurement values indicative of a presence and/or concentration of each of said constituents,
wherein said list is indicative of the group a operating mode belongs to.

9. The method of any of the preceding claims wherein, upon receiving two different selections of operating modes from different sources, said two operating modes are executed alternatingly.

10. A portable sensor device comprising
a gas sensor (30),
a control and processing means (45) for controlling said gas sensor (30) and for processing data from said gas sensor (30),
an application programming interface means (47) that provides access to said control and processing means (45),
**characterized in that** said control and processing means (45) comprises
an operating mode list provider (54) for providing a list of a plurality of different operating modes and providing said list to said application programming interface means (47),
- wherein each of said operating modes is indicative of a method for controlling said gas sensor (30) and wherein at least some of said operating modes are indicative of different methods for controlling said gas sensor (30) and/or
- wherein each of said operating modes is indicative of a method for processing said data from said gas sensor (30) and wherein at least some of said operating modes are indicative of different methods for processing said data from said gas sensor (30), and
an operating mode controller (52) adapted to receive a selection of an operating mode through said application programming interface means (47) and to control said gas sensor (30) and/or to process said data from said gas sensor (30).

11. The sensor device of claim 10 further comprising an interface (23) for connecting said device to a remote resource (25) over a network (24), wherein said list comprises, for each operating mode, an indicator if said access is required.

12. The sensor device of any of the claims 10 or 11 further comprising a second sensor (31), in particular a humidity sensor, in addition to said gas sensor (30), wherein said operating mode controller (52) is adapted to combine, for at least one selected operating mode, data from said second sensor (31) with data from said gas sensor (30), and to not combine, for at least one other selected operating mode, the data from said second sensor (31) with the data from said gas sensor (30).

13. The sensor device of any of the claims 10 to 12, wherein said gas sensor (30) comprises a metal-oxide layer (35a, 35b, 35c, 35d) and a heater (38) for heating said metal-oxide layer (35a, 35b, 35c, 35d).

14. The sensor device of any of the claims 10 to 14 comprising a plurality of gas sensors, in particular of metal-oxide gas sensors.

15. A control and processing means for being used in the method or the portable sensor device of any of the preceding claims, **characterized in that** said control and processing means comprises
an operating mode list provider (54) for providing a list of a plurality of different operating modes and providing said list to an application programming interface means (47),
- wherein each of said operating modes is indicative of a method for controlling said gas sensor (30) and wherein at least some of said operating modes are indicative of different methods for controlling said gas sensor (30) and/or
- wherein each of said operating modes is indicative of a method for processing said data from said gas sensor (30) and wherein at least some of said operating modes are indicative of different methods for processing said data from said gas sensor (30), and
an operating mode controller (52) responsive to a selection of a operating mode made through said application programming interface means (47) in order to control said gas sensor (30) and/or to process said data from said gas sensor (30) in accordance with the selected operating mode.

16. The control and processing means of claim 15 being a software library.

## Patentansprüche

1. Ein Verfahren zum Betrieb einer tragbaren Sensorvorrichtung, wobei die tragbare Sensorvorrichtung
einen Gassensor (30),
ein Steuerungs- und Verarbeitungsmittel (45) zum Steuern des Gassensors (30) und zur Verarbeitung von Daten vom Gassensor (30); und
ein Anwendungsprogramm-Schnittstellenmittel API (47) welches Zugang zum Steuerungs- und Verarbeitungsmittel (45) bereitstellt,
umfasst,
wobei das Verfahren durch die Schritte gekennzeichnet ist:
a) Bereitstellen einer Liste einer Vielzahl von unterschiedlichen Betriebsmodi und Bereitstellen der Liste durch die API,
- wobei jeder der Betriebsmodi ein Verfahren zur Steuerung des Gassensors (30) bezeichnet und wobei mindestens einige der Betriebsmodi verschiedene Verfahren zur Steuerung des Gassensors (30) bezeichnen und/oder
- wobei jeder der Betriebsmodi ein Verfahren zur Verarbeitung von Daten vom Gassensors (30) bezeichnet und wobei mindestens einige der Betriebsmodi verschiedene Verfahren zur Verarbeitung von Daten vom Gassensor (30) bezeichnen, und
b) Empfangen einer Auswahl von einem der Betriebsmodi, durchgeführt mittels der Anwendungsprogramm-Schnittstellenmittel (47), und Steuern des Gassensors (30) und/oder Verarbeiten der Daten vom Gassensor (30) gemäss dem ausgewählten Betriebsmodus.

2. Das Verfahren nach einem der vorangehenden Ansprüche, wobei mindestens einige der Betriebsmodi Zugang der Sensorvorrichtung zu Ressourcen (25) über ein Netzwerk (24) benötigen, wobei die Liste für jeden Betriebsmodus einen Indikator umfasst, ob Zugang benötigt wird.

3. Das Verfahren nach einem der vorangehenden Ansprüche, wobei die Liste auf eine erwartete Dauer für eine Messung in jedem Betriebsmodus hinweist.

4. Das Verfahren nach einem der vorangehenden Ansprüche, wobei mindestens einige der Betriebsmodi verschiedene Temperaturen oder Temperaturprofile bezeichnen, bei welchen der Gassensor (30) betrieben werden muss.

5. Das Verfahren nach einem der vorangehenden Ansprüche, wobei die tragbare Vorrichtung einen zweiten Sensor (31) zusätzlich zum Gassensor (30) umfasst, wobei mindestens einer der Betriebsmodi auf eine Kombination von Daten vom zweiten Sensor (31) mit Daten vom Gassensor (30) im Schritt b) hinweist, wobei mindestens ein anderer Betriebsmodus nicht auf eine Kombination von Daten vom zweiten Sensor (31) mit Daten vom Gassensor (30) im Schritt b) hinweist.

6. Das Verfahren nach Anspruch 5, wobei der zweite Sensor (31) ein Feuchtigkeitssensor ist.

7. Das Verfahren nach einem der vorangehenden Ansprüche, wobei die tragbare Vorrichtung eine Vielzahl (35a, 35b, 35c, 35d) von Gassensoren umfasst, insbesondere von Metalloxid-Gassensoren, wobei mindestens einige der Betriebsmodi auf eine unterschiedliche Verwendung von Signalen von der Vielzahl (35a, 35b, 35c, 35d) von Gassensoren im Schritt b) hinweisen.

8. Das Verfahren nach einem der vorangehenden Ansprüche, wobei eine erste Gruppe der Betriebsmodi zur Detektion einzelner Gase oder Gasmischungen und zur Generierung eines Messwerts, der auf eine Anwesenheit und/oder Konzentration des einzelnen Gases oder der Gasmischung hinweist, ausgestaltet ist, während
eine zweite Gruppe der Betriebsmodi zum Analysieren einer Gaszusammensetzung mit mehreren Bestandteilen und zur Ausgabe von Messwerten, die auf eine Anwesenheit und/oder Konzentration von jedem der Bestandteile hinweisen, ausgestaltet ist,
wobei die Liste auf die Gruppe hinweist, zu welcher der Betriebsmodus gehört.

9. Das Verfahren nach einem der vorangehenden Ansprüche, wobei nach dem Empfang zweier unterschiedlicher Selektionen von Betriebsmodi von verschiedenen Quellen die zwei Betriebsmodi abwechselnd ausgeführt werden.

10. Eine tragbare Sensorvorrichtung, umfassend
einen Gassensor (30),
ein Steuerungs- und Verarbeitungsmittel (45) zum Steuern des Gassensors (30) und zur Verarbeitung von Daten vom Gassensor (30), und
ein Anwendungsprogramm-Schnittstellenmittel (47) welches Zugang zum Steuerungs- und Verarbeitungsmittel (45) bereitstellt,
umfasst,
**dadurch gekennzeichnet dass** die Steuerungs- und Verarbeitungsmittel (45)
einen Anbieter einer Betriebsmodusliste (54) zum Bereitstellen einer Liste einer Vielzahl von unterschiedlichen Betriebsmodi und Bereitstellen der Liste an das Anwendungsprogramm-Schnittstellenmittel (47),
- wobei jeder der Betriebsmodi ein Verfahren zur Steuerung des Gassensors (30) bezeichnet und wobei mindestens einige der Betriebsmodi verschiedene Verfahren zur Steuerung des Gassensors (30) bezeichnen und/oder
- wobei jeder der Betriebsmodi ein Verfahren zur Verarbeitung von Daten vom Gassensors (30) bezeichnet und wobei mindestens einige der Betriebsmodi verschiedene Verfahren zur Verarbeitung von Daten vom Gassensors (30) bezeichnen, und
eine Betriebsmodussteuerung (52), ausgestaltet zum Empfangen einer Auswahl eines Betriebsmoduses durch die Anwendungsprogramm-Schnittstellenmittel (47) und zum Steuern des Gassensors (30) und/oder zum Verarbeiten der Daten vom Gassensor (30),
umfassen.

11. Die Sensorvorrichtung nach Anspruch 10, weiter umfassend eine Schnittstelle (23) zum Verbinden der Vorrichtung mit einer fernen Ressource (25) über ein Netzwerk (24), wobei die Liste einen Indikator für jeden Betriebsmodus umfasst, ob der Zugang benötigt wird.

12. Die Sensorvorrichtung nach einem der Ansprüche 10 oder 11, weiter umfassend einen zweiten Sensor (31), insbesondere einen Feuchtigkeitssensor, zusätzlich zum Gassensor (30), wobei die Betriebsmodussteuerung (52) zum Kombinieren von Daten vom zweiten Sensor (31) mit Daten vom Gassensor (30) für mindestens einen ausgewählten Betriebsmodus ausgestaltet ist, und nicht zum Kombinieren von Daten vom zweiten Sensor (31) mit Daten vom Gassensor (30) für mindestens einen ausgewählten Betriebsmodus ausgestaltet ist.

13. Die Sensorvorrichtung nach einem der Ansprüche 10 bis 12, wobei der Gassensor (30) eine Metalloxidschicht (35a, 35b, 35c, 35d) und eine Heizung (38) zum Heizen der Metalloxidschicht (35a, 35b, 35c, 35d) umfasst.

14. Die Sensorvorrichtung nach einem der Ansprüche 10 bis 14, umfassend eine Vielzahl von Gassensoren, insbesondere von Metalloxid-Gassensoren.

15. Ein Steuerungs- und Verarbeitungsmittel zur Verwendung im Verfahren oder in der tragbaren Sensorvorrichtung nach einem der vorangehenden Ansprüche, **dadurch gekennzeichnet, dass** das Steuerungs- und Verarbeitungsmittel
einen Anbieter einer Betriebsmodusliste (54) zum Bereitstellen einer Liste einer Vielzahl von unterschiedlichen Betriebsmodi und Bereitstellen der Liste an das Anwendungsprogramm-Schnittstellenmittel (47),
- wobei jeder der Betriebsmodi ein Verfahren zur Steuerung des Gassensors (30) bezeichnet und wobei mindestens einige der Betriebsmodi verschiedene Verfahren zur Steuerung des Gassensors (30) bezeichnen und/oder
- wobei jeder der Betriebsmodi ein Verfahren zur Verarbeitung von Daten vom Gassensors (30) bezeichnet und wobei mindestens einige der Betriebsmodi verschiedene Verfahren zur Verarbeitung von Daten vom Gassensors (30) bezeichnen, und
eine Betriebsmodussteuerung (52), ausgestaltet zum Empfangen einer Auswahl eines Betriebsmoduses durch die Anwendungsprogramm-Schnittstellenmittel (47) und zum Steuern des Gassensors (30) und/oder zum Verarbeiten der Daten vom Gassensor (30) in Übereinstimmung mit dem ausgewählten Betriebsmodus,
umfasst.

16. Das Steuerungs- und Verarbeitungsmittel nach Anspruch 15, welches eine Softwarebibliothek ist.

## Revendications

1. Un procédé pour opérer un dispositif de capteur portable, ledit dispositif capteur portable comprenant
un capteur de gaz (30),
un moyen de commande et traitement (45) pour commander ledit capteur de gaz (30) et pour traiter des données dudit capteur de gaz (30), et
un moyen d'interface de programmation d'applications API (47) qui fournit un accès au moyen de commande et traitement (45),
ledit procédé étant **caractérisé par** les étapes
a) de fournir une liste d'une pluralité des modes d'opération différents et de fournir ladite liste par ledit API,
- chacun desdits modes d'opération indiquant un procédé pour commander ledit capteur de gaz (30) et au moins quelques-uns des modes d'opération indiquant des procédés différents pour commander ledit capteur de gaz (30) et/ou
- chacun desdits modes d'opération indiquant un procédé pour traiter lesdites données dudit capteur de gaz (30) et au moins quelques-uns des modes d'opération indiquant des procédés différents pour traiter lesdites données dudit capteur de gaz (30), et
b) de recevoir une sélection d'un desdits modes d'opération, effectué par moyen d'interface de programmation d'applications (47), et de commander ledit capteur de gaz (30) et/ou de traiter lesdites données dudit capteur de gaz (30) selon le mode d'opération sélectionnée.

2. Le procédé selon l'une des revendications précédentes, au moins quelques-uns des modes d'opération nécessitant un accès du dispositif de capteur à des ressources (25) via un réseau (24), ladite liste comprenant pour chaque mode d'opération un indicateur si ledit accès est nécessaire.

3. Le procédé selon l'une des revendications précédentes, ladite liste indiquant une durée anticipée pour une mesure dans chaque mode d'opération.

4. Le procédé selon l'une des revendications précédentes, au moins quelques-uns des modes d'opération indiquant des différentes températures ou profiles de température en présence desquelles/desquels le capteur de gaz (30) doit être opéré.

5. Le procédé selon l'une des revendications précédentes, ledit dispositif mobile comprenant un deuxième capteur (31), en plus dudit capteur de gaz (30), au moins un desdits modes d'opération indiquant une combinaison dans l'étape b) des données dudit deuxième capteur (31) avec des données dudit capteur de gaz (30), au moins un autre desdits modes d'opération indiquant dans l'étape b) aucune combinaison des données dudit deuxième capteur (31) avec des données dudit capteur de gaz (30).

6. Le procédé selon la revendication 5, ledit deuxième capteur (31) étant un capteur d'humidité.

7. Le procédé selon l'une des revendications précédentes, ledit dispositif mobile comprenant une pluralité (35a, 35b, 35c, 35d) de capteurs de gaz, particulièrement de capteurs de gaz d'oxyde de métal, au moins quelques-uns des modes d'opération indiquant une utilisation différente des signaux de ladite pluralité (35a, 35b, 35c, 35d) de capteurs de gaz dans ladite étape b).

8. Le procédé selon l'une des revendications précédentes,
un premier groupe desdits modes d'opération étant conçu à détecter des gaz ou des mélanges des gaz spécifiques et à générer une valeur de mesure indiquant une présence et/ou une concentration dudit gaz ou de ladite mélange de gaz spécifique,
alors qu'un deuxième groupe desdits modes d'opération est conçu à analyser une composition de gaz avec des différents composants et à fournir des valeurs de mesure indiquant une présence et/ou une concentration de chacun desdits composants,
ladite liste indiquant le groupe auquel un mode d'opération appartient.

9. Le procédé selon l'une des revendications précédentes, les deux modes d'opération étant exécutés de manière alternante après avoir reçu deux différentes sélections de modes d'opération de différentes sources.

10. Un dispositif de capteur portable, comprenant
un capteur de gaz (30),
un moyen de commande et traitement (45) pour commander ledit capteur de gaz (30) et pour traiter des données dudit capteur de gaz (30), et
un moyen d'interface de programmation d'applications (47) qui fournit un accès au moyen de commande et traitement (45),
**caractérisé en ce que** ledit moyen de commande et traitement (45) comprend
un fournisseur de liste de modes d'opération (54) pour fournir une liste d'une pluralité des modes d'opération différents et pour fournir ladite liste audit moyen d'interface de programmation d'applications (47),
- chacun desdits modes d'opération indiquant un procédé pour commander ledit capteur de gaz (30) et au moins quelques-uns des modes d'opération indiquant des procédés différents pour commander ledit capteur de gaz (30) et/ou
- chacun desdits modes d'opération indiquant un procédé pour traiter lesdites données dudit capteur de gaz (30) et au moins quelques-uns des modes d'opération indiquant des procédés différents pour traiter lesdites données dudit capteur de gaz (30), et
une commande de mode d'opération (52) adaptée à recevoir une sélection d'un desdits modes d'opération, effectué par le moyen d'interface de programmation d'applications (47), et à commander ledit capteur de gaz (30) et/ou à traiter lesdites données dudit capteur de gaz (30).

11. Le dispositif de capteur selon la revendication 10, comprenant en outre une interface (23) pour connecter ledit dispositif à une source distante (25) via un réseau (24), la liste comprenant pour chaque. mode d'opération un indicateur si ledit accès est nécessaire.

12. Le dispositif de capteur selon l'une des revendications 10 ou 11, comprenant en outre un deuxième capteur (31), particulièrement un capteur d'humidité, en plus dudit capteur de gaz (30), ladite commande de mode d'opération (52) étant adaptée à combiner pour au moins un mode d'opération sélectionné des données dudit deuxième capteur (31) avec des données dudit capteur de gaz (30), et à ne pas combiner pour au moins un autre mode d'opération sélectionné des données dudit deuxième capteur (31) avec des données dudit capteur de gaz (30).

13. Le dispositif de capteur selon l'une des revendications 10 à 12, ledit capteur de gaz (30) comprenant une couche d'oxyde de métal (35a, 35b, 35c, 35d) et un chauffage (38) pour chauffer ladite couche d'oxyde de métal (35a, 35b, 35c, 35d).

14. Le dispositif de capteur selon l'une des revendications 10 à 14, comprenant une pluralité de capteurs de gaz, particulièrement de capteurs de gaz à oxyde de métal.

15. Un moyen de commande et traitement pour être utilisé dans le procédé ou le dispositif de capteur portable selon l'une des revendications précédentes, **caractérisé en ce que** le moyen de commande et traitement comprend
un fournisseur de liste de modes d'opération (54) pour fournir une liste d'une pluralité des modes d'opération différents et pour fournir ladite liste audit moyen d'interface de programmation d'applications (47),
- chacun desdits modes d'opération indiquant un procédé pour commander ledit capteur de gaz (30) et au moins quelques-uns des modes d'opération indiquant des procédés différents pour commander ledit capteur de gaz (30) et/ou
- chacun desdits modes d'opération indiquant un procédé pour traiter lesdites données dudit capteur de gaz (30) et au moins quelques-uns des modes d'opération indiquant des procédés différents pour traiter lesdites données dudit capteur de gaz (30), et
une commande de mode d'opération (52) réceptive à une sélection d'un mode d'opération, effectué par le moyen d'interface de programmation d'applications (47), afin de commander ledit capteur de gaz (30) et/ou de traiter lesdites données dudit capteur de gaz (30), en concordance avec le mode d'opération sélectionné.

16. Le moyen de commande et traitement selon la revendication 15 étant une bibliothèque logicielle.
